# EUROPEAN PATENT SPECIFICATION

(11) **EP 0 490 998 B1**
(45) Date of publication and mention of the grant of the patent: **06.12.1995**
(21) Application number: 90914638.3
(22) Date of filing: 07.09.1990
(51) Int. Cl.: A61K 47/48

(54) **TRANSFERRIN RECEPTOR SPECIFIC ANTIBODY-NEUROPHARMACEUTICAL AGENT CONJUGATES**
TRANSFERRINREZEPTOR-SPEZIFISCHE ANTIKÖRPER - NEUROPHARMAZEUTISCHES MITTEL-KONJUGATE
CONJUGUES ANTICORPS-AGENT NEUROPHARMACEUTIQUE SPECIFIQUES AU RECEPTEUR DE TRANSFERRINE

(30) Priority: 07.09.1989 US 404089
(43) Date of publication of application: 24.06.1992
(73) Proprietor: ALKERMES, INC., Cambridge, MA 02139 (US)
(72) Inventor: FRIDEN, Phillip, Bedford, MA 01730 (US)
(74) Representative: Holdcroft, James Gerald, Dr.
(86) International application number: PCT/US90/05077
(87) International publication number: WO 91/03259

(56) References cited:
- EP-A- 0 094 844
- EP-A- 0 175 560
- EP-A- 0 286 418
- EP-A- 0 305 967
- EP-A- 0 324 625
- EP-A- 0 327 169
- EP-A- 0 328 147
- EP-A- 0 336 383
- WO-A-86/01409
- WO-A-88/07365
- US-A- 4 292 425
- US-A- 4 545 985
- File Server STN Karlsruhe, File Chemical Abstracts, Abstract no. CA108(17): 142953k; V. Raso et al.: "Monensin is obligatory for the cytotoxic action of a disulfide linked methotrexate-anti-transferrin receptor conjugate"
- File Server STN Karlsruhe, File Chemical Abstracts, Abstract no. CA108(9): 68464h; M.J. Bjorn et al.: "Immunotoxins to the murine transferrin receptor: intracavitary therapy of mice bearing syngeneic peritoneal tumors"
- File Server STN Karlsruhe, File Biosis Accession no. 88:73068 and Biological Abstract 85:39367; M.J. Smyth et al.: "The mode of action of methotrexate-monoclonal antibody conjugates"
- Nature, vol. 312, 8 November 1984, (GB); W.A. Jefferies et al.: "Transferrin receptor on endothelium of brain capillaries", pages 162-3
- Endocrine Reviews, vol. 7, no. 3, 1986, The Endocrine Society, Baltimore, US; W.M. Pardridge: "Receptor-mediated peptide transport through the bloodbrain barrier", pages 314-330
- Proc. Natl. Acad. Sci. USA, vol. 78, no. 7, July 1981, Washington, D.C., US; R. Sutherland et al.: "Ubiquitous cell-surface glycoprotein on tumor cells is proliferation-associated receptor for transferrin", pages 4515-4519
- Biochemical and Biophysical Research Communications, volume 2, no. 3, 1981, 15 October 1981, Academic Press Inc., New York, US; W.-C. Shen et al.: "Cis-aconityl spacer between daunomycin and macromolecular carriers: a model of PH-sensitive linkage releasing drug from a lysosomotropic conjugate", pages 1048-1054

## Description

### Background

The capillaries that supply blood to the tissues of the brain constitute the blood brain barrier (Goldstein et al. (1986) Scientific American 255:74-83; Pardridge, W.M. (1986) Endocrin. Rev. 7:314-330). The endothelial cells which form the brain capillaries are different from those found other tissues in the body. Brain capillary endothelial cells are joined together by tight intercellular junctions which form a continuous wall against the passive movement of substances from the blood to the brain. These cells are also different in that they have few pinocytic vesicles which in other tissues allow somewhat unselective transport across the capillary wall. Also lacking are continuous gaps or channels running through the cells which would allow unrestricted passage.

The blood-brain barrier functions to ensure that the environment of the brain is constantly controlled. The levels of various substances in the blood, such as hormones, amino acids and ions, undergo frequent small fluctuations which can be brought about by activities such as eating and exercise (Goldstein et al, cited supra). If the brain were not protected by the blood brain barrier from these variations in serum composition, the result could be uncontrolled neural activity.

The isolation of the brain from the bloodstream is not complete. If this were the case, the brain would be unable to function properly due to a lack of nutrients and because of the need to exchange chemicals with the rest of the body. The presence of specific transport systems within the capillary endothelial cells assures that the brain receives, in a controlled manner, all of the compounds required for normal growth and function. In many instances, these transport systems consist of membrane-associated receptors which, upon binding of their respective ligand, are internalized by the cell (Pardridge, W.M., cited supra). Vesicles containing the receptor-ligand complex then migrate to the abluminal surface of the endothelial cell where the ligand is released.

The problem posed by the blood-brain barrier is that, in the process of protecting the brain, it excludes many potentially useful therapeutic agents. Presently, only substances which are sufficiently lipophilic can penetrate the blood-brain barrier (Goldstein et al, cited supra; Pardridge, W.M., cited supra). Some drugs can be modified to make them more lipophilic and thereby increase their ability to cross the blood brain barrier. However, each modification has to be tested individually on each drug and the modification can alter the activity of the drug. The modification can also have a very general effect in that it will increase the ability of the compound to cross all cellular membranes, not only those of brain capillary endothelial cells.

EP-A-0 328 147 is directed to anthracycline immunoconjugates which are targeted and subsequently incorporated into particular cell types. The immunological components of these immunoconjugates are antibodies such as anti-transferrin receptor antibodies. The eventually incorporated immunoconjugates are cytocidal to the target cells. The specific immunoconjugate is a cytocidal anthracycline derivative.

According to one aspect, the present invention provides use of an antibody-neuropharmaceutical agent conjugate when the antibody is reactive with a transferrin receptor, for the manufacture of a medicament for delivering the neuropharmaceutical agent across the blood brain barrier to the brain of a host, under conditions whereby binding of the antibody to transferrin receptors present on brain capillary endothelial cells occurs and the neuropharmaceutical agent is transferred across the blood brain barrier in a pharmaceutically active form.

According to another aspect, the invention provides a conjugate for delivering a neuropharmaceutical agent across the blood brain barrier comprising an antibody reactive with a transferrin receptor linked to a growth factor, whereby the conjugate transports the growth factor across the blood brain barrier when administered in vivo.

Still further, the invention provides a conjugate for delivering a neuropharmaceutical agent across the blood brain barrier comprising an antibody reactive with a transferrin receptor linked to a nucleotide analog, whereby the conjugate transports the nucleotide analog across the blood brain barrier when administered in vivo.

Still further, the invention provides use of an antibody-neuropharmaceutical agent conjugate wherein the antibody is reactive with a transferrin receptor, for the manufacture of a medicament for treating a host afflicted with a disease associated with a neurological disorder, under conditions whereby binding of the antibody to the transferrin receptor present on a brain capillary endothelial cell occurs and the neuropharmaceutical agent is transferred across the blood brain barrier in a pharmaceutically active form.

Still further, the invention provides use of a ligand-neuropharmaceutical agent conjugate wherein the ligand is reactive with a transferrin receptor, for the manufacture of a medicament for delivering a neuropharmaceutical agent across the blood brain barrier to the brain of a host under conditions whereby binding of the ligand to transferrin receptors present on brain capillary endothelial cells occurs and the neuropharmaceutical agent is transferred across the blood brain barrier in a pharmaceutically active form.

Presently available means for delivering therapeutic agents to the brain are limited in that they are invasive. The present invention is non-invasive and can utilize readily available antibodies reactive with a transferrin receptor as carriers for neuropharmaceutical agents. This is advantageous in that the antibodies are capable of transporting neuropharmaceutical agents across the blood brain barrier without being susceptible to premature release of the neuropharmaceutical agent prior to reaching the brain-side of the blood brain barrier. Further, if the therapeutic activity of the agent to be delivered to the brain is not altered by the addition of a linker, a noncleavable linker can be used to link the neuropharmaceutical agent to the antibody.

### Description of the Drawings

Figure 1 is a graphic representation of rat brain uptake of ¹⁴C-labelled murine monoclonal antibody (OX-26) to rat transferrin receptor in rats where the percent injected dose of radiolabelled antibody per brain and per 55 »l of blood is plotted versus time post-injection.

Figure 2 is a histogram illustrating time dependent changes in the disposition of radiolabelled OX-26 between brain parenchyma and vasculature.

Figure 3 is a histrogram illustrating the enhanced delivery of methotrexate across the blood-brain barrier when administered as a conjugate with OX-26.

Figure 4 illustrates in three histograms (A, B and C) the distribution in the brain of both the antibody and the AZT components of an OX-26-AZT conjugate.

Figure 5 is a histogram illustrating the experimental results of delivery of a protein, horseradish peroxidase, across the blood-brain barrier in rat brains in the form of a conjugate with OX-26.

Figure 6 is a histogram illustrating the experimental results of delivering soluble CD4 to rat brain parenchyma using CD4 in the form of a conjugate with OX-26.

Figure 7 is a histogram illustrating the biodistribution of antibody 128.1 and control IgG in a cynomolgous monkey.

### Detailed Description

The method for delivering a neuropharmaceutical agent across the blood brain barrier to the brain of a host comprises administering to the host a therapeutically effective amount of an antibody-neuropharmaceutical agent conjugate wherein the antibody is reactive with a transferrin receptor present on a brain capillary endothelial cell. The method is conducted under conditions whereby the antibody binds to the transferrin receptor on the brain capillary endothelial cell and the neuropharmaceutical agent is transferred across the blood brain barrier in a pharmaceutically active form.

The host can be an animal susceptible to a neurological disorder (i.e., an animal having a brain). Examples of hosts include mammals such as humans, domestic animals (e.g., dog, cat, cow or horse), mice and rats.

The neuropharmaceutical agent can be an agent having a therapeutic or prophylatic effect on a neurological disorder or any condition which affects biological functioning of the central nervous system. Examples of neurological disorders include cancer (e.g. brain tumors), Autoimmune Deficiency Syndrome (AIDS), epilepsy, Parkinson's disease, multiple sclerosis, neurodegenerative disease, trauma, depression, Alzheimer's disease, migraine, pain, or a seizure disorder. Classes of neuropharmaceutical agents which can be used in this invention include proteins, antibiotics, adrenergic agents, anticonvulsants, small molecules, nucleotide analogs, chemotherapeutic agents, anti-trauma agents, peptides and other classes of agents used to treat or prevent a neurological disorder. Examples of proteins include CD4 (including soluble portions thereof), growth factors (e.g. nerve growth factor and interferon), dopamine decarboxylase and tricosanthin. Examples of antibiotics include amphotericin B, gentamycin sulfate and pyrimethamine. Examples of adrenergic agents (including blockers) include dopamine and atenolol. Examples of chemotherapeutic agents include adriamycin, methotrexate, cyclophosphamide, etoposide, and carboplatin. An example of an anticonvulsant which can be used is valproate and an anti-trauma agent which can be used is superoxide dimutase. Examples of peptides would be somatostatin analogues and enkephalinase inhibitors. Nucleotide analogs which can be used include azido thymidine (hereinafter AZT), dideoxy Inosine (ddI) and dideoxy cytidine (ddC).

Serum transferrin is a monomeric glycoprotein with a molecular weight of 80,000 daltons that binds iron in the circulation and transports it to the various tissues(Aisen et al. (1980) Ann. Rev. Biochem. 49:357-393; MacGillivray et al. (1981) J. Biol. Chem. 258:3543-3553). The uptake of iron by individual cells is mediated by the transferrin receptor, an integral membrane glycoprotein consisting of two identical 95,000 dalton subunits that are linked by a disulfide bond. The number of receptors on the surface of a cell appears to correlate with cellular proliferation, with the highest number being on actively growing cells and the lowest being on resting and terminally differentiated cells. Jeffries et al (Nature Vol. 312 (November 1984) pp. 167-168) used monoclonal antibodies to show that brain capillary endothelial cells have transferrin receptors on their cell surface.

Antibodies which can be used within this invention are reactive with a transferrin receptor. The term antibody is intended to encompass both polyclonal and monoclonal antibodies. The preferred antibody is a monoclonal antibody reactive with a transferrin receptor. The term antibody is also intended to encompass mixtures of more than one antibody reactive with a transferrin receptor (e.g., a cocktail of different types of monoclonal antibodies reactive with a transferrin receptor). The term antibody is further intended to encompass whole antibodies, biologically functional fragments thereof, chimeric antibodies comprising portions from more than one species, bifunctional antibodies, etc. Biologically functional antibody fragments which can be used are those fragments sufficient for binding of the antibody fragment to the transferrin receptor to occur.

The chimeric antibodies can comprise portions derived from two different species (e.g., human constant region and murine binding region). The portions derived from two different species can be joined together chemically by conventional techniques or can be prepared as a single contiguous protein using genetic engineering techniques. DNA encoding the proteins of both portions of the chimeric antibody can be expressed as a contiguous protein.

The term transferrin receptor is intended to encompass the entire receptor or portions thereof. Portions of the transferrin receptor include those portions sufficient for binding of the receptor to an anti-transferrin receptor antibody to occur.

Monoclonal antibodies reactive with at least a portion of the transferrin receptor can be obtained (e.g., OX-26, B3/25 (Omary et al. (1980) Nature 286,888-891), T56/14 (Gatter et al. (1983) J. Clin. Path. 36 539-545; Jefferies et al. Immunology (1985) 54:333-341), OKT-9 (Sutherland et al. (1981) Proc. Natl. Acad. Sci. USA 78:4515-4519), L5.1 (Rovera, C. (1982) Blood 59:671-678), 5E-9 (Haynes et al.(1981) J. Immunol. 127:347-351), RI7 217 (Trowbridge et al. Proc. Natl. Acad. Sci. USA 78:3039 (1981) and T58/30 (Omary et al. cited supra)or can be produced using conventional somatic cell hybridization techniques (Kohler and Milstein (1975) Nature 256, 495-497). A crude or purified protein or peptide comprising at least a portion of the transferrin receptor can be used as the immunogen. An animal is vaccinated with the immunogen to obtain an anti-transferrin receptor antibody-producing spleen cells. The species of animal immunized will vary depending on the species of monoclonal antibody desired. The antibody producing cell is fused with an immortalizing cell (e.g. myeloma cell) to create a hybridoma capable of secreting anti-transferrin receptor antibodies. The unfused residual antibody-producing cells and immortalizing cells are eliminated. Hybridomas producing the anti-transferrin receptor antibodies are selected using conventional techniques and the selected anti-tranferrin receptor antibody producing hybridomas are cloned and cultured.

Polyclonal antibodies can be prepared by immunizing an animal with a crude or purified protein or peptide comprising at least a portion of a transferrin receptor. The animal is maintained under conditions whereby antibodies reactive with a transferrin receptor are produced. Blood is collected from the animal upon reaching a desired titer of antibodies. The serum containing the polyclonal antibodies (antisera) is separated from the other blood components. The polyclonal antibody-containing serum can optionally be further separated into fractions of particular types of antibodies (e.g. IgG, IgM).

The neuropharmaceutical agent can be linked to the antibody using standard chemical conjugation techniques. Generally, the link is made via an amine or a sulfhydryl group. The link can be a cleavable link or non-cleavable link depending upon whether the neuropharmaceutical agent is more effective when released in its native form or whether the pharmaceutical activity of the agent can be maintained while linked to the antibody. The determination of whether to use a cleavable or non-cleavable linker can be made without undue experimentation by measuring the activity of the drug in both native and linked forms or for some drugs can be determined based on known activities of the drug in both the native and linked form.

For some cases involving the delivery of proteins or peptides to the brain, release of the free protein or peptide may not be necessary if the biologically active portion of the protein or peptide is uneffected by the link. As a result, antibody-protein or antibody peptide conjugates can be constructed using noncleavable linkers. Examples of such proteins or peptides include CD4, superoxide dismutase, interferon, nerve growth factor, tricosanthin, dopamine decarboxylase, somatostatin analogues and enkephalinase inhibitors. Terms such as "CD4" are used herein to include modified versions of the natural molecule, such as soluble CD4, truncated CD4, etc. Examples of non-cleavable linker systems which can be used in this invention include the carbodiimide (EDC), the sulfhydrylmaleimide, the N-succinimidyl-3-(2-pyridyldithio) propionate (SPDP; Pharmacia), and the periodate systems. In the carbodiimide system, a water soluble carbodiimide reacts with carboxylic acid groups on proteins and activates the carboxyl group. The carboxyl group is coupled to an amino group of the second protein. The result of this reaction is a noncleavable amide bond between two proteins.

In the sulfhydryl-maleimide system, a sulfhydryl group is introduced onto an amine group of one of the proteins using a compound such as Traut's reagent. The other protein is reacted with an NHS ester (such as gamma-maleimidobutyric acid NHS ester (GMBS)) to form a maleimide derivative that is reactive with sulfhydryl groups. The two modified proteins are then reacted to form a covalent linkage that is noncleavable.

SPDP is a heterofunctional crosslinking reagent that introduces aliphatic thiol groups into either the monoclonal antibody or the neuropharmaceutical agent. The thiol group reacts with an amine group forming a non-cleavable bond.

Periodate coupling requires the presence of oligosaccharide groups on either the carrier or the protein to be delivered. If these groups are available on the protein to be delivered (as in the case of horseradish peroxidase (HRP)), an active aldehyde is formed on the protein to be delivered which can react with an amino group on the carrier. It is also possible to form active aldehyde groups from the carbohydrate groups present on antibody molecules. These groups can then be reacted with amino groups on the protein to be delivered generating a stable conjugate. Alternatively, the periodate oxidized antibody can be reacted with a hydrazide derivative of a protein to be delivered which will also yield a stable conjugate.

The antibody-protein conjugate can also be produced as a contiguous protein using genetic engineering techniques. Gene constructs can be prepared comprising DNA encoding the anti-transferrin receptor antibody fused to DNA encoding the protein to be delivered across the blood brain barrier. The protein can be expressed as a contiguous molecule containing both an antibody portion and a neuropharmaceutical agent portion.

Cleavable linkers can be used to link neuropharmaceutical agents which are to be deposited in the brain or when a non-cleavable linker alters the activity of a neuropharmaceutical agent.

Acid labile linkers include cis-aconitic acid, cis-carboxylic alkadienes, cis-carboxylic alkatrienes, and poly-maleic anhydrides. Other cleavable linkers are linkers capable of attaching to primary alcohol groups. Examples of neuropharmaceutical agents which can be linked via a cleavable link include AZT, ddI, ddC, adriamycin, amphotericin B, pyrimethamine, valproate, methotrexate, cyclophosphamide, carboplatin and superoxide dimutase. The noncleavable linkers used generally to link proteins to the antibody can also be used to link other neuropharmaceutical agents to the antibody.

In addition to covalent binding, conjugates can be formed employing non-covalent bonds, such as those formed with bifunctional antibodies, ionic bonds, hydrogen bonds, hydropholic interactions, etc. The important consideration is that the conjugate bond be strong enough to result in passage of the conjugate through the blood-brain barrier.

The antibody-neuropharmaceutical agent conjugates can be administered orally, by subcutaneous or other injection, intravenously, intramuscularly, parenternally, transdermally, nasally or rectally. The form in which the conjugate is administered (e.g., capsule, tablet, solution, emulsion) will depend at least in part on the route by which it is administered.

A therapeutically effective amount of an antibody-neuropharmaceutical agent conjugate is that amount necessary to significantly reduce or eliminate symptoms associated with a particular neurological disorder. The therapeutically effective amount will be determined on an individual basis and will be based, at least in part, on consideration of the individuals's size, the severity of symptoms to be treated, the result sought, the specific antibody, etc. Thus, the therapeutically effective amount can be determined by one of ordinary skill in the art employing such factors and using no more than routine experimentation.

Although the description above focuses on antibodies, any protein which interacts with the extracellular domain of the transferrin receptor, including the ligand binding site, could potentially serve as a vehicle for the delivery of drugs across the blood-brain barrier. In addition to anti-transferrin receptor antibodies, this would include transferrin, the ligand which binds to the receptor, and any transferrin derivatives which retain receptor-binding activity. In fact, any ligand which binds to the transferrin receptors could potentially be employed.

The present invention will be further illustrated by the following specific examples.

### EXAMPLE 1- In Vitro Binding of Murine Monoclonal Antibodies to Human Brain Endothelial Cells

Two murine monoclonal antibodies, B3/25 and T58/30, described by Trowbridge (U.S. Patent 4,434,156 issued February 28, 1984, and Nature Vol. 294, pp. 171-173 (1981)), the contents of both are hereby incorporated by reference, which recognize the human transferrin receptor were tested for their ability to bind to human brain capillary endothelial cells. Hybridoma cell lines which produce B3/25 and T58/30 antibodies were obtained from the American Type Culture Collection (ATTC) in Rockville, Maryland, and grown in DMEM medium supplemented with 2.0 mM glutamine, 10.0 mM HEPES (pH 7.2), 100 »M non-essential amino acids and 10% heat-inactivated fetal calf serum. The hybridoma cultures were scaled-up in 225 cm² T-flasks for the production of milligram quantities of IgG antibody. The hybridoma supernatants were concentrated 50x using vacuum dialysis and applied to a protein-A sepharose column using the BioRad MAPS buffer system. Purified antibody was eluted from the column, dialyzed against 0.1 M sodium phosphate (pH 8.0), concentrated and stored in aliquots at -20°C.

Primary cultures of human brain endothelial cells were grown in flat-bottom 96-well plates until five days post-confluency. The cells were then fixed using 3.0% buffered formalin and the plate blocked with 1.0% bovine serum albumin (BSA) in Dulbecco's phosphate buffered saline (DPBS). Aliquots (100 »l) of the B3/25 or T58/30 antibodies, either in the form of culture supernatants or purified protein, were then added to the wells (antibody concentrations were in the range of 1-50 »g/ml). Antibody which had specifically bound to the fixed cells was detected using a biotin-labeled polyclonal goat-anti-mouse IgG antisera followed by a biotinylated horseradish peroxidase (HRP)/avidin mixture (Avidin Biotin Complex technique). Positive wells were determined using a Titertek Multiscan Enzyme Linked Immunosorbent Assay (ELISA) plate reader. The results showed that both antibodies bind to human brain capillary endothelial cells with the T58/30 antibody exhibiting a higher level of binding.

These same antibodies were also tested for binding to human brain capillaries using sections of human brain tissue that were fresh frozen (without fixation), sectioned on a cryostat (section thickness was 5-20 »m), placed on glass slides and fixed in acetone (10 minutes at room temperature). These sections were then stored at -20°C prior to use.

The slides containing the human brain sections were allowed to come to room temperature prior to use. The sections were then rehydrated in DPBS and incubated in methanol containing 0.3% H₂O₂ to block endogenous peroxidase activity. The sections were blocked for fifteen minutes in a solution containing 0.2% non-fat dry milk and 0.2% methylmannopyranoside. B3/25 and T58/30 antibodies, purified as discussed above, were applied to the sections at a concentration of 5-50 »g/ml and incubated at room temperature for one to two hours. Antibody that specifically bound to the tissue was detected using the Avidin-Biotin Complex (ABC) technique as described above for the ELISA assay. Staining of capillaries in the human brain sections was observed with both the B3/25 and T58/30 antibodies. The T58/30 antibody also displayed some binding to the white matter of the brain cortex.

### EXAMPLE 2- In-Vitro Binding of Murine Monoclonal Antibody OX-26 to Rat Transferrin Receptor

The OX-26 murine antibody, which recognizes the rat transferrin receptor, has been shown in vivo to bind to brain capillary endothelial cells (Jeffries et al., cited supra). The murine hybridoma line which produces the OX-26 murine antibody was obtained and the hybridoma cell line was grown in RPMI 1640 medium supplemented with 2.0 mM glutamine and 10% heat-inactivated fetal calf serum. The OX-26 antibody was purified using the affinity chromatography technique described in Example 1. The purified antibody was tested in vitro as described for the anti-human transferrin receptor antibodies in Example 1 to determine whether it would bind to brain capillaries in fresh frozen, acetone-fixed rat brain sections. The results showed that the OX-26 anti-transferrin receptor antibody did bind to capillaries in rat brain sections in vitro.

### EXAMPLE 3- In-Vivo Binding of OX-26 Murine Monoclonal Antibody to Rat Transferrin Receptor

### Dose Range

The anti-rat transferrin receptor antibody OX-26 was tested in vivo by injecting purified antibody (purification as described in Example 1) into female Sprague-Dawley rats (100-150 gm) via the tail vein. Prior to injection, the rats were anesthetized with halothane. The samples, ranging from 2.0 mg to 0.05 mg of antibody/rat were injected into the tail vein in 400 »l aliquots. All doses were tested in duplicate animals. One hour post-injection, the animals were sacrificed and perfused through the heart with DPBS to clear the blood from the organs. Immediately after the perfusion was completed, the brain was removed and quick frozen in liquid nitrogen. The frozen brain was then sectioned (30-50 »m) on a cryostat and the sections placed on glass microscope slides. The brain sections were air dried at room temperature one to two hours before fixation in acetone (10 minutes at room temperature). After this treatment the sections could be stored at -20°C.

The OX-26 antibody was localized in the brain sections using immunohistochemistry as described above for the in vitro experiments in Example 1. The addition of the primary antibody was unnecessary in that it is present in the brain sections. The results indicated that the OX-26 antibody binds to rat brain capillary endothelial cells and that doses of as little as 50 »g result in detectable levels of antibody in the brain using the methods described herein. Doses above 0.5 mg did not appear to show significantly more antibody binding to the endothelial cells, suggesting that the sites for antibody binding may be saturated. No specific binding to capillary endothelium was detected in the liver, kidney, heart, spleen or lung.

A non-specific antibody of the same subclass as OX-26 (IgG 2a) was also tested in vivo to show that the binding of OX-26 to rat brain endothelial cells that has been observed is specific to the OX-26 antibody. 0.5 mg of the control antibody was injected per rat as described above. The results indicate that the staining pattern observed with the OX-26 antibody is specific to that antibody.

### Time Course

After establishing that the OX-26 antibody is detectable in the rat brain capillaries after in vivo administration, the time frame in which this binding occurred was determined. Using 0.5 mg of purified OX-26 antibody as the standard dose, brain sections taken from animals sacrificed 5 minutes, 15 minutes, 1 hour, 2 hours, 4 hours, 8 hours and 24 hours post-injection were examined for the presence of OX-26 antibody. All doses were administered in 400 »l aliquots and each time point was tested in duplicate animals. Samples were injected and the rats were processed at the various times post-injection as described above in the dose range section.

The results showed that the OX-26 antibody can be detected in or on the rat brain capillary endothelial cells as early as five minutes and as late as 24 hours post-injection. At 4 and 8 hours post-injection, the staining pattern of the antibody is very punctate suggesting that the antibody has accumulated in pericytes.

### EXAMPLE 4- The Use of a Conjugate of OX-26 Murine Monoclonal Antibody for Tranferring Horseradish Peroxidase Across the Blood Brain Barrier

Horseradish Peroxidase (HRP; 40 kD) was chosen as a compound to be delivered to the brain because it is similar in size to several therapeutic agents and it can be easily detected in the brain using an enzymatic assay. HRP was conjugated to the OX-26 antibody using a non-cleavable periodate linkage and the ability of the antibody to function as a carrier of compounds to the brain was examined. The antibody conjugate was tested in vivo to determine if the antibody could deliver HRP to the brain.

The antibody (10 mg) was first dialyzed overnight against 0.01 M sodium bicarbonate (pH 9.0). The HRP (10 mg) was dissolved in 2.5 ml deionized water, 0.1 M sodium periodate (160 »l) was added and the mixture was incubated for five minutes at room temperature. Ethylene glycol (250 »l) was added to the HRP solution followed by an additional five minute incubation. This solution was then dialyzed overnight against 1.0 mM sodium acetate buffer (pH 4.4). To the dialyzed OX-26 antibody (2.0 ml, 5.08 mg/ml) was added 200 »l of 1.0 M sodium bicarbonate buffer, pH 9.5 and 1.25 ml of the dialyzed HRP solution. This mixture was incubated in the dark for two hours followed by the addition of 100 »l of 10 mg/ml sodium borohydride. The resulting mixture was incubated two additional hours in the dark at 4°C. The protein was precipitated from the solution by the addition of an equal volume of saturated ammonium sulfate and resuspended in a minimal volume of water. Free antibody was removed from the mixture by chromatography on a concanavalin A-sepharose column (a column which binds HRP and the HRP-antibody conjugate and allows the free antibody to pass through). The free HRP was removed by chromatography on a protein A-sepharose column which retains the antibody-HRP conjugate. The final product had an HRP/antibody ratio of 4/1.

A time course experiment identical to that described in Example 3 was performed using the antibody-HRP conjugate. The antibody-HRP conjugate (0.5 mg) was injected in a 400 »l aliquot/rat. The animals were sacrificed at the various times post-injection and the brains processed as described above in Example 3. The antibody HRP conjugate was localized in the brain either by staining for antibody immunohistochemically as described in Example 1 or by directly staining the brain sections for the presence of HRP. To detect HRP, the slides were first allowed to come to room temperature before incubating in methanol for thirty minutes. The brain sections were then washed in DPBS and reacted with 3,3'-diamino benzidine (DAB), the substrate for HRP. The results showed that the OX-26 antibody HRP conjugate binds to rat brain capillary endothelial cells in a manner identical to that of the unconjugated antibody. The punctate staining 4-8 hours after injection which was seen with the antibody alone is also seen with the antibody conjugate, suggesting that the conjugate can also be going into the pericytes on the abluminal side of the blood brain barrier. Taken together, these results indicate that the OX-26 antibody can deliver a protein molecule of at least 40 KD to the brain.

### EXAMPLE 5- The In-Vivo Delivery of Adriamycin to the Brain by Murine Monoclonal Antibody OX-26

A non-cleavable linker system similar to that used in Example 4, was used to couple the chemotherapeutic drug adriamycin to the OX-26 antibody. The availability of antibodies that can detect adriamycin as well as the system previously described in Example 1 for detecting the antibody carrier allowed the use of immunohistochemical techniques for monitoring the localization of the antibody carrier as well as the delivery of adriamycin to the brain.

To conjugate adriamycin to the antibody, the drug (10 mg in 0.5 ml DPBS) was oxidized by the addition of 200 »l of 0.1 M sodium periodate. This mixture was incubated for one hour at room temperature in the dark. The reaction was quenched by the addition of 200 »l of ethylene glycol followed by a five minute incubation. The OX-26 antibody (5.0 mg in 0.5 ml of carbonate buffer (pH 9.5)) was added to the oxidized adriamycin and incubated at room temperature for one hour. Sodium borohydride (100 »l of 10 mg/ml) was added and the mixture was incubated for an additional two hours at room temperature. The free adriamycin was separated from the OX-26 antibody-adriamycin conjugate by chromatography on a PD-10 column. The adriamycin/OX-26 antibody ratio within the conjugate was 2/1. for this particular batch of conjugate.

The effectiveness of the OX-26 antibody as a carrier for delivering adriamycin to the brain was determined by administering 0.5 mg of the antibody-adriamycin conjugate in a 400 »l aliquot per rat by injection via the tail vein. One hour post-injection, the rat was sacrificed and the brain processed as described in Example 1. All injections were performed in duplicate. As a control, 400 »g of free adriamycin in a 400 »l aliquot was also injected into a rat. Immunohistochemistry was used to detect both the carrier OX-26 antibody and the adriamycin in the rat brain sections. In the case of adriamycin, polyclonal rabbit anti-adriamycin antisera was applied to the sections followed by a biotinylated goat anti-rabbit IgG antisera. This was then followed by the addition of a biotinylated HRP/avidin mixture and enzymatic detection of HRP.

The results indicate that both the OX-26 antibody and the conjugated adriamycin localized to the rat brain capillary endothelial cells after in vivo administration. There is no evidence that free adriamycin binds to brain capillary endothelial cells or enters the brain.

An adriamycin-OX-26 conjugate coupled via a carbodiimide linkage was also synthesized (drug/antibody ratio of 10/1) and tested in vivo. The results of this experiment were essentially identical to that obtained with the periodate-linked antibody-drug conjugate. In both cases, staining for the antibody carrier was quite strong and was visualized in the capillaries in all areas of the brain. This staining was evenly distributed along the capillaries. Staining for adriamycin was less intense but again was seen in capillaries throughout the brain. Some punctate staining was observed which suggests accumulation in pericytes which lie on the brain side of the blood-brain barrier.

### EXAMPLE 6- In Vivo Delivery of Methotrexate to the Brain by Murine Monoclonal Antibody OX-26.

A noncleavable carbodiimide linkage was used to couple methotrexate to the OX-26 murine monoclonal antibody. A system analogous to that described in Example 5 was used to monitor the delivery of both the methotrexate and the carrier antibody to the brain capillary endothelial cells.

Methotrexate was coupled to murine monoclonal antibody OX-26 via its active ester. Briefly, 81 mg (0.178 mM) of methotrexate (Aldrich) was stirred with 21 mg (0.182 mM) of N-hydroxysuccinimide (Aldrich) in 3 ml of dimethylformamide (DMF) at 4°C. Ethyl-3-dimethylaminopropyl-carbodiimide (180 mg;EDC;0.52mM) was added to this solution and the reaction mixture was stirred overnight. The crude ester was purified from the reaction by-products by flash chromatography over silica gel 60 (Merck) using a solution of 10% methanol in chloroform as an eluant. The purified active ester fractions were pooled and concentrated to dryness. The ester was dissolved in 1 ml of DMF and stored at -20°C until use. 50 mg (50%) of active ester was recovered as determined by A₃₇₂(ₑ₃₇₂₌7200).

A solution of OX-26 containing 2.1 mg (14 nmoles) of antibody in 0.9 ml of 0.1 M phosphate (pH 8.0) was thawed to 4 °C. To this stirred antibody solution was added 1.4 »L (140 nmoles) of the active ester prepared as described above. After 16 hours at 4°C, the mixture was chromatographed over Sephadex PD-10 column (Pharmacia) using phosphate buffered saline (PBS) to separate conjugate from free drug. The fractions containing the antibody-methotrexate conjugate were pooled. Antibody and drug concentration were determined spectrophotometrically as described by Endo et al. (Cancer Research (1988) 48:3330-3335). The final conjugate contained 7 methotrexates/antibody.

The ability of the OX-26 monoclonal antibody to deliver methotrexate to the rat brain capillary endothelial cells was tested in vivo by injecting 0.2 mg of conjugate (in 400 »l) into each of two rats via the tail vein. The animals were sacrificed one hour post-injection and the brains processed for immunohistochemistry as described in Example 1. To detect methotrexate in the brain, a rabbit antisera raised against methotrexate was used as the primary antibody. A biotinylated goat-anti-rabbit antisera in conjunction with a mixture of biotinylated HRP and avidin was then used to visualize methotrexate in the rat brain. The carrier antibody was detected as described previously.

The results of these experiments indicate that methotrexate in the form of a conjugate with OX-26 does accumulate along or in the capillary endothelial cells of the brain. The staining observed for methotrexate is comparable in intensity to that seen for the carrier. The staining appears to be in all areas of the brain and is evenly distributed along the capillaries.

### EXAMPLE 7- Antibody Fragments

The Fc portion of the OX-26 murine monoclonal antibody was removed to determine whether this would alter its localization to or uptake by the rat brain capillary endothelial cells. F(ab)₂ fragments of OX-26 were produced from intact IgG's via digestion with pepsin. A kit available from Pierce Chemical Co. contains the reagents and protocols for cleaving the antibody to obtain the fragments . The F(ab')₂ fragment (0.2 mg doses) in 400 »l aliquots were injected into rats via the tail vein. A time course experiment identical to that done with the intact antibody (Example 2) was then performed. F(ab')₂ fragment was detected immunohistochemically using a goat anti-mouse F(ab')₂ antisera followed by a biotinylated rabbit anti-goat IgG antisera. A biotinylated HRP/avidin mixture was added and the antibody complex was visualized using an HRP enzymatic assay. The results indicated that the F(ab)₂ fragment of the OX-26 antibody bound to the capillary endothelial cells of the rat brain.

### EXAMPLE 8 - Measurement of OX-26 in Brain Tissue

To quantitate the amount of OX-26 which accumulates in the brain, radioactively-labelled antibody was injected into rats via the tail vein. Antibodies were labelled with either ¹⁴C-acetic anhydride or ³H-succinimidyl proprionate essentially as described in Kummer, U., Methods in Enzymology, 121: 670-678 (1986), Mondelaro, R.C., and Rueckert, R.R., J. of Biological Chemistry, 250: 1430-1421 (1975), hereby incorporated by reference. For all experiments, the radiolabelled compounds were injected as a 400 »l bolus into the tail vein of female Sprague-Dawley rats (100-125 gms) under Halothane anesthesia and the animals were sacrificed at the appropriate time post-injection using a lethal dose of anesthetic. A ³H-labelled IgG2a control antibody was co-injected with the ¹⁴C-labelled OX-26 to serve as a control for non-specific radioactivity in the brain due to residual blood. At the appropriate time post-injection, animals were sacrificed and the brains were removed immediately and homogenized in 5 ml of 0.5% sodium dodecylsulfate using an Omni-mixer. An aliquot of the homogenate was incubated overnight with 2 ml of Soluene 350 tissue solubilizer prior to liquid scintillation counting. All data were collected as disintegrations per minute (dpm). Blood samples were centrifuged to pellet red blood cells (which do not display significant binding of radiolabelled materials) and the radioactivity in an aliquot of serum determined using liquid scintillation counting.

The amount of antibody associated with the brain was determined at various times post-injection to examine the pharmocokinetics of brain uptake. In addition, the amount of labelled antibody in the blood was measured so that the rate of clearance from the bloodstream could be determined. This information was also used to calculate the amount of radioactivity in the brain due to blood contamination, which was then subtracted from the total to give the amount of antibody that is specifically associated with the brain.

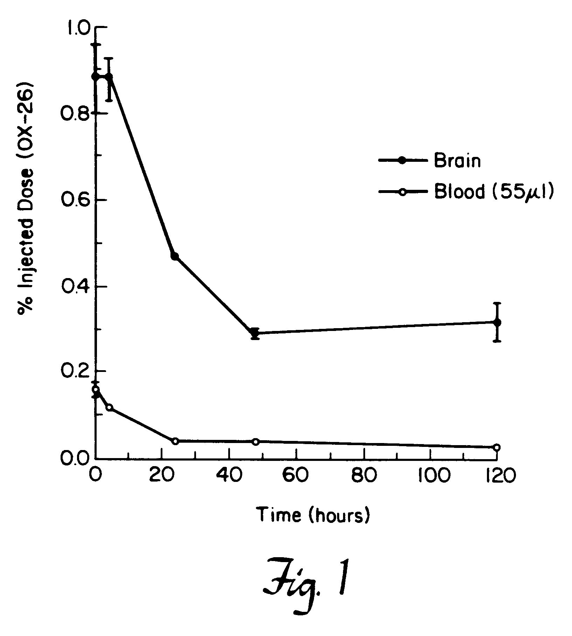

A peak level of ¹⁴C-labelled OX-26 corresponding to approximately 0.9% of the injected dose was reached in the brain between 1 and 4 hours post-injection as illustrated in Figure 1 (with the values shown as means plus or minus standard error of measurement (SEM) and N=3 rats per time point). The amount of radioactivity associated with the brain decreased steadily from 4 to 48 hours post-injection, at which point it leveled off at approximately 0.3% of the injected dose. The accumulation of OX-26 in the brain was significantly reduced by the addition of unlabelled monoclonal antibody (0.5 or 2.0 mg in the bolus injection) . As an additional control, a ³H- IgG2a control antibody was co-injected with the ¹⁴C-OX-26. The control antibody did not accumulate in the brain and represented the blood contamination of the brain.

In contrast to the levels in the brain, the blood level of OX-26 dropped quite dramatically immediately after injection such that by 1 hour post-injection, the percent of injected dose in 55 »l of blood (the volume of blood associated with the brain) was approximately 0.16% as illustrated in Figure 1. This corresponds to a value of approximately 20% of the injected dose in the total blood volume of the rat. Extraction of total IgG from serum followed by polyacrylmide gel electrophoresis (PAGE) and autoradiography did not reveal detectable levels of OX-26 degradation indicating that the antibody remains intact in the blood as long as 48 hours after injection.

### EXAMPLE 9 - Distribution of OX-26 in Brain Parenchyma and Capillaries

To demonstrate that anti-transferrin receptor antibody accumulates in the brain parenchyma, homogenates of brains taken from animals injected with labelled OX-26 were depleted of capillaries by centrifugation through dextran to yield a brain tissue supernatant and a capillary pellet. Capillary depletion experiments followed the procedure of Triguero, et al., J. of Neurochemistry, 54: 1882-1888 (1990), hereby incorporated by reference. As for the brain up take experiments of Example 8, the radiolabelled compounds were injected as a 400 »l bolus into the tail vein of female Srague-Dawley rats (100-125 gm) under Halothane anesthesia and the animals were sacrificed at the appropriate time post-injection using a lethal dose of anesthetic. A ³H-labelled IgG 2a control antibody was co-injected with the ¹⁴C- labelled OX-26 to serve as a control for non-specific radioactivity in the brain due to residual blood. After sacrifice, the brains were removed and kept on ice. After an initial mincing, the brains were homogenized by hand (8-10 strokes) in 3.5 m of ice cold physiologic buffer (100 mM NaCl, 4.7 mM KCl, 2.5 mM CaCl₂, 1.2 mM KH₂PO₄, 1.2 mM MgSO₄, 14.5 mM HEPES, 10 mM D-glucose, pH 7.4). Four ml of 26% dextran solution in buffer was added and homogenization was continued (3 strokes). After removing an aliquot of the homogenate, the remainder was spun at 7200 rpm in a swinging bucket rotor. The resulting supernatant was carefully removed from the capillary pellet. The entire capillary pellet and aliquots of the homogenate and supernatant were incubated overnight with 2 ml of Soluene 350 prior to liquid scintillation counting. This method removes greater than 90% of the vasculature from the brain homogenate (Triguero et al., cited supra).

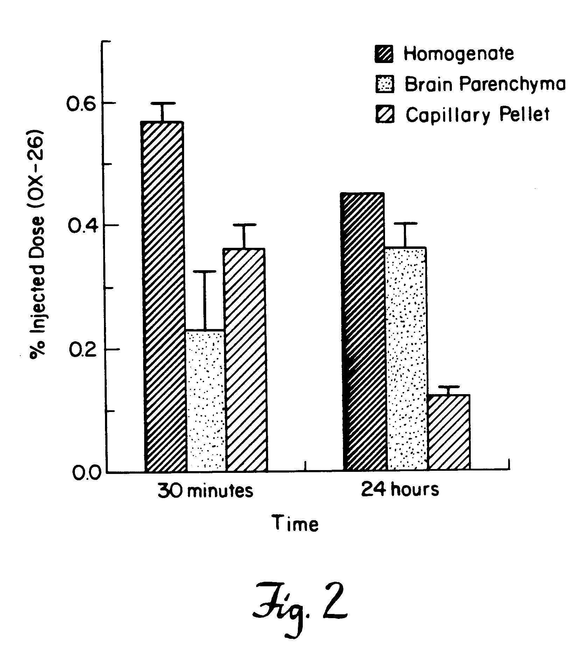

A comparison of the relative amounts of radioactivity in the different brain fractions as a function of time indicates whether transcytosis of the labelled antibody has occurred. The amount of OX-26 in total brain homogenate, the brain parenchyma fraction and the brain capillary fraction at an early time (30 minutes) and a later time (24 hours) post-injection is illustrated in Figure 2. The values in Figure 2 are shown as means±SEM with N=3 rats per time point. At the 30 minute time point, more of the radiolabelled antibody is associated with the capillary fraction than with the brain parenchyma fraction (0.36% of the injected dose (%ID) and 0.23% ID, respectively). By 24 hours post-injection, the distribution is reversed and the majority of the radioactivity (0.36% ID) in the parenchymal fraction as compared to the capillary fraction (0.12% ID). The redistribution of the radiolabelled OX-26 from the capillary fraction to the parenchyma fraction is consistent with the time dependent migration of the anti-transferrin receptor antibody across the blood- brain barrier.

### EXAMPLE 10 - Distribution of an OX-26-methotrexate Conjugate in Brain Parenchyma and Capillaries

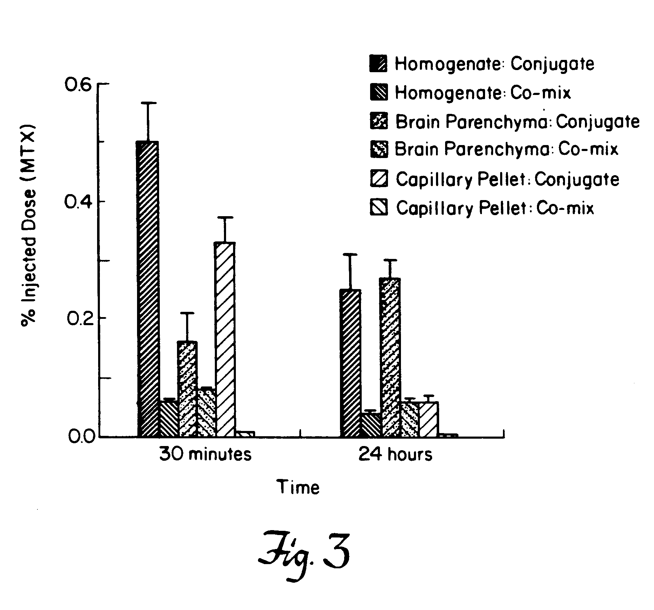

Capillary depletion studies following the procedures described in Example 9 were performed with an OX-26-methotrexate (MTX) conjugate linked via a gamma- hydrazid as described in Kralovec, et al., J. of Medicinal Chem., 32: 2426-2431 (1989), hereby incorporated by reference, in which the MTX moiety was labelled with ³H. As with unconjugated antibody, the amount of label in the capillary fraction at 30 minutes post-injection is greater than the parenchyma fraction (approximately 2 fold as illustrated in Figure 3, with the data expressed as means±SEM and N=3 rats per time point). This distribution changes over time such that by 24 hours post- injection, approximately 4.5-fold more of the labelled MTX is in the brain parenchyma than in the capillaries. These results are consistent to those obtained with unconjugated antibody and, again, suggest that these compounds cross the blood-brain barrier.

To ensure that these results were not due to contaminating amounts of free ³H-MTX or ³H-MTX that had been cleaved from the conjugate after injection, a co-mix of labelled drug and antibody was injected into rats and a capillary depletion experiment performed. The amount of ³H-MTX in the different brain fraction is significantly lower for the co-mix as compared to the conjugate (as much as 47 fold in the case of the capillary fraction at 30 minutes post-injection as illustrated in Figure 3). The ³H-MTX and the co-mix also does not show the change in distribution of the label between the different brain fractions over time as was seen with the antibody-MTX conjugate or antibody alone. These results demonstrate that delivery of ³H-MTX across the blood-brain barrier to the brain parenchyma is greatly enhanced by the conjugation of the drug to the anti-transferrin receptor antibody OX-26.

### EXAMPLE 11 - Distribution of OX-26-AZT in Brain Parenchyma and Capillaries

Capillary depletion studies following the procedures of Example 9 were performed with an OX-26-AZT conjugate using a pH-sensitive succinate linker. These studies employed a dual-labelled conjugate in which the AZT was ¹⁴C-labelled and the antibody carrier was ³H-labelled. The use of such a conjugate allowed independent monitoring of the disposition of both the antibody and AZT within the brain.

The linker was synthesized as follows. Succinic anhydride was used to acylate the AZT by reacting equimolar amounts of these two compounds for 3 hours at room temperature under argon in the presence of dimethylaminopyridine and sodium bisulfate in freshly distilled pyridine. The product was isolated by chromatography on a DEAE sephadex A50 column run with a triethylammonium bicarbonate buffer. The succinate derivative of AZT was activated at the carboxyl group as the NHS ester by reaction with equimolar amounts of N-hydroxysuccinimide and dicyclohexylcarbodiimide (DCC) in freshly distilled THF at 4°C fo 2 hours. The product was purified by flash chromatography on silica gel. The resulting NHS-ester of AZT-succinate was used to acylate amine groups on OX-26, resulting in an AXT-OX-26 conjugate. A 15-fold molar excess of AZT-NHS ester was reacted with OX-26 in HEPES buffer overnight at 4°C. The antibody-drug conjugate was isolated from free drug on a PD-10 column. The molar ratio of drug to antibody was 7:1. These studies employed a dual-labelled conjugate in which the AZT was ¹⁴C-labelled and the antibody carrier was ³H-labelled.

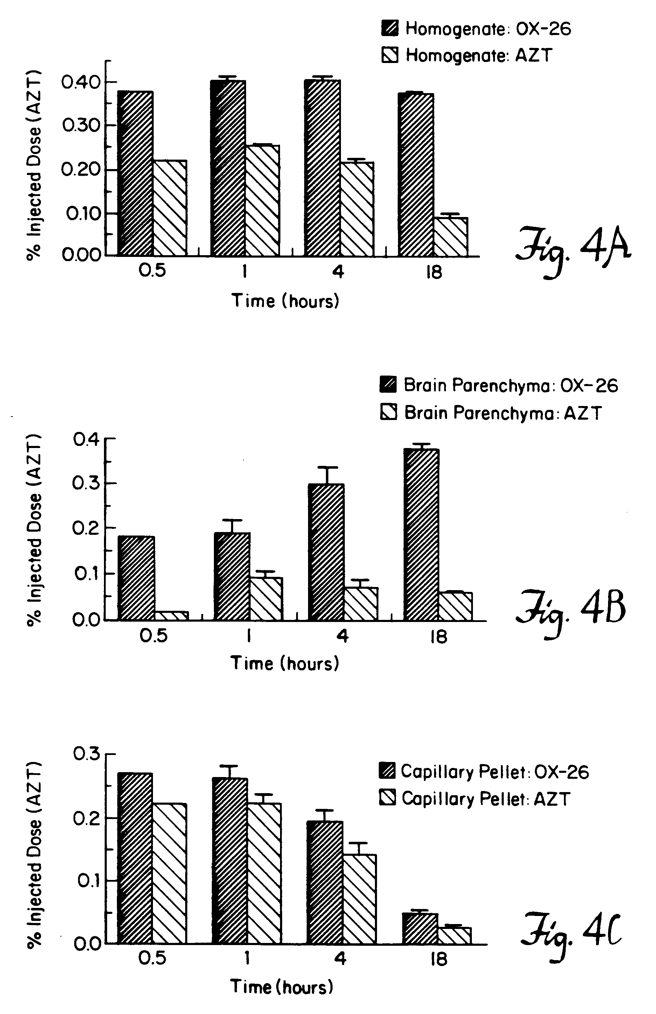

Similar levels of OX-26 and AZT are seen in the capillary fraction of the brain and these levels decrease with time, suggesting that the materials are not being retained by the capillary endotheliel cells as illustrated in Figure 4c. As the levels of OX-26 in the capillary fraction decrease, the levels in the parenchyma fraction increase, indicating that the antibody is migrating from the capillaries to the parenchyma in a time-dependent manner as illustrated in Figure 4b. In contrast, the levels of AZT in the brain parenchyma do not rise significantly, suggesting that the majority of the drug is released in the endotheliel cells and is not transported across the blood-brain barrier. The levels of OX-26 and AZT remained similar in unfractionated homogenates over time as illustrated in Figure 4a. The data in Figure 4 are expressed as means±SEM with N=3 rats per time point. These results indicate that the linker is cleaved within the endothelial cells and may represent a method for delivering compounds to those cells.

### EXAMPLE 12 - Distribution of OX-26-Horseradish Peroxidase (HRP) in Brain Parenchyma and Capillaries

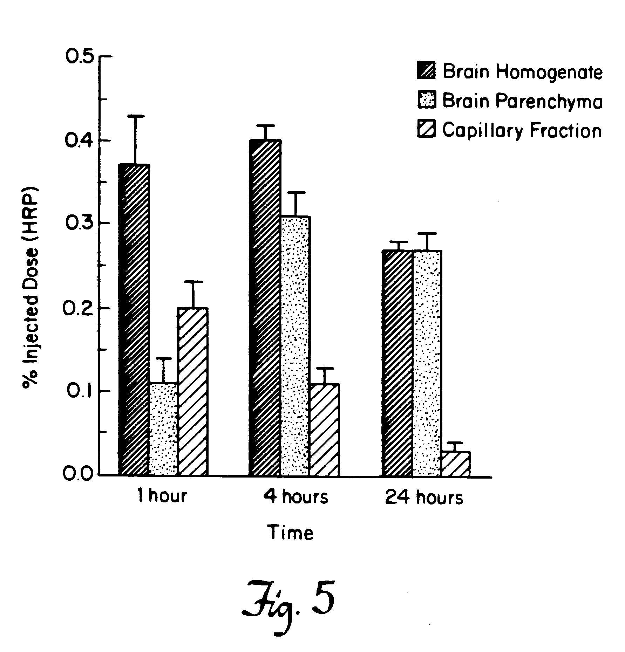

Capillary depletion studies following the proceduresdescribed for OX-26 in Example 9 were preformed with a ³H-labelled OX-26-HRP conjugate that was prepared using a non-cleavable periodate linkage as described in Example 4. The tritium label was distributed between the antibody and the HRP portion of the conjugate. At 1 hour post-injection, the majority of the radioactivity associated with the brain is in the capillary fraction as illustrated in Figure 5. The data in Figure 5 are expressed as means±SEM with N=3 rats per time point. By 4 hours post-injection, the distribution of radioactivity associated with the brain changed such that the majority is in the fraction which represents the brain parenchyma. At 24 hours post-injection, essentially all of the ³H-labelled OX-26-HRP conjugate is in the parenchyma fraction of the brain indicating that the material has crossed the blood- brain barrier. Similar results were obtained in experiments in which only the HRP portion of the conjugate was radiolabelled.

The percent of injected dose of the OX-26-HRP conjugate that reaches the brain is somewhat lower than that for antibody alone or the OX-26-MTX conjugate. This is most likely due to the presence of 2 to 3 40 kD HRP molecules attached to each carrier and that these "passenger" molecules are randomly attached to the carrier. Due to this, many of the HRP passengers may be attached to the antibody in such a way as to interfere with antigen recognition. This problem can be alleviated by directing the attachment of the passenger to regions of the carrier removed from critical functional domains.

### EXAMPLE 13 - Distribution of OX-26-CD4 in Brain Parenchyma and Capillaries

A soluble form of CD4, consisting of amino acids 1-368, was conjugated to OX-26 using a linkage that directed the attachment of the CD4 to the carbohydrate groups located in the Fc portion of the antibody. By directing the site of attachment in this way, the chance that the passenger molecules will interfere with antibody-antigen recognition is lessened. The linkage between the proteins was achieved by first introducing a sulfhydryl group onto CD4 using SATA (N-Succinimidyl S-acetylthioacetate), a commercially available compound. A hydrizid derivative of SDPD, another commerical cross-linking agent, was attached to OX-26 via carbohydrate groups on the antibody. Reaction of the two modified proteins gives rise to a disulfide-linked conjugate.

More specifically, the linkage between the proteins was achieved by first introducing a sulfhydryl group onto CD4 using N-succinimidyl S-acetylthioacetate (SATA), a commercially available compound. A 4-fold molar excess of SATA was added to 5 mg of CD4 in 0.1 M sodium phosphate buffer containing 3 mM EDTA (pH 7.5). This mixture was reacted at room temperature in the dark for 30 minutes. Unreacted starting materials were removed by passage over a PD-10 column. A hydrizid derivative of SPDP, another commercially available cross-linking agent, was attached to OX-26 via carbohydrate groups on the antibody. Ten milligrams of OX-26 in 2.0 ml of 0.1 M sodium acetate, 0.15 M sodium chloride (pH 5.0) was reacted with a 1000-fold molar excess of sodium periodate for 1 hour at 4°C in the dark. Unreacted starting materials were removed by passage over a PD-10 column. The oxidized antibody was reacted with a 30-fold molar excess of hydrazido-SPDP overnight at 4°C with stirring. Reaction of the two modified proteins gives rise to a disulfide-linked conjugate. One tenth volume of 0.5 M hydroxylamine was added to the thioacetylated CD4 (CD4-DATA) and derivatized antibody was then added such that the ratio of CD4 to antibody was 7.5:1. This mixture was reacted at room temperature in the dark for 2 hours. Conjugate was purified by running the reaction mixture over a protein A column followed by a CD4 affinity column.

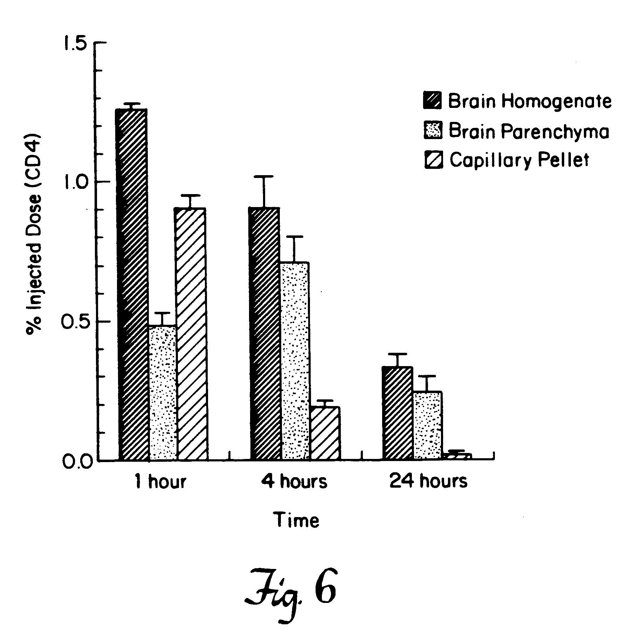

Capillary depletion experiments following the procedures described in Example 9 with OX-26 were performed with an OX-26-CD4 conjugate in which only the CD4 portion was ³H-labelled. Time dependent changes in the distribution of the labelled conjugate between the capillary and parenchyma fractions of the brain which are consistent with transcytosis across the blood-brain barrier were observed as illustrated in Figure 6. The data in Figure 6 are expressed as means±SEM with N=3 rats per time point.

### EXAMPLE 14 - Biodistribution and Brain Uptake of Anti-Human Transferrin Receptor Antibodies in Cynomolgous Monkeys

A collection of 32 murine monoclonal antibodies which recognize various epitopes on the human transferrin receptor were examined for reactivity with brain capillary endothelial cells in sections from human, monkey (cynomolgous), rat and rabbit brain samples by the immunohistochemical methods described in Example 1. These antibodies were obtained from Dr. Ian Trowbridge of the Salk Institute, La Jolla, CA. All 32 antibodies displayed some reactivity with human brain endotheliel cells. Two antibodies reacted very weakly with rabbit brain capillaries and none reacted with rat. While 21 of the antibodies reacted with monkey brain capillaries, only 2 displayed strong reactivity comparable to that seen with human brain capillaries. These 2 antibodies are herewithin referred to as 128.1 and 335.2.

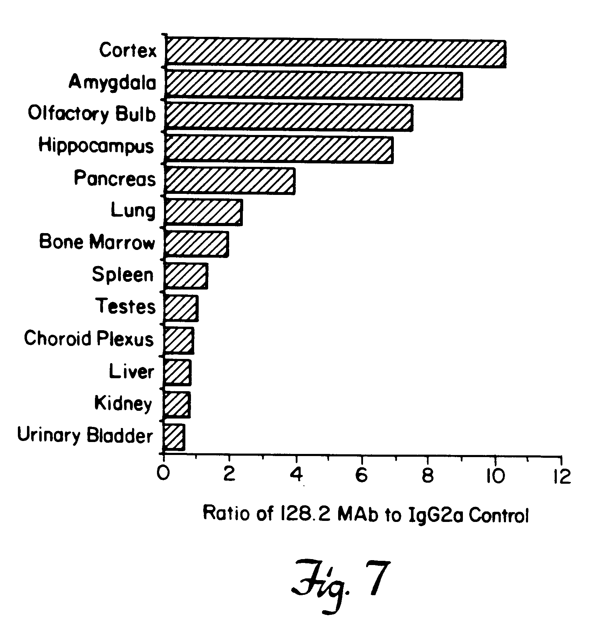

These antibodies were used to determine the tissue distribution and blood clearance of the ¹⁴C-labelled anti-human transferrin receptor antibodies 128.1 and 335.2 in 2 male cynomolgous monkeys. 128.1 or 335.2 was administered concurrently with a ³H-labelled control IgG to one of the monkeys with an intravenous catheter. During the course of the study, blood samples were collected to determine the clearance of the antibodies from the circulation. At 24 hours post-injection, the animals were euthanized and selected organs and representative tissues were collected for the determination of isotope distribution and clearance by combustion. In addition, samples from different regions of the brain were processed as described for the capillary depletion experiments in Example 9 to determine whether the antibodies had crossed the blood-brain barrier. The results of the capillary depletion experiments were performed on samples from the cortex, frontal cortex, cerebellum and striatum. All samples had greater than 90% of the 128.1 or 335.2 in the brain parenchyma, suggesting that the antibodies crossed the blood-brain barrier. The levels of the control antibody in the same samples were from 5 to 10-fold lower. Using the average brain homogenate value for dpm/G tissue, the percent injected dose of 128.1 in the whole brain is approximately 0.2-0.3%. This compares to a value of 0.3-0.5% for OX-26 in the rat at 24 hours post-injection. A comparison of the ratios of 128.1 to the control antibody for various organs is illustrated in Figure 7. Similar results were obtained for 335.2. These results suggest that 128.1 is preferentially taken up by the brain as compared to control antibody. For the majority of organs and tissues tested, the ratio of 128.1 to control is less than 2.

## Claims (Claims for the following Contracting State(s): AT, BE, CH, DE, DK, FR, GB, IT, LI, LU, NL, SE)

1. Use of an antibody-neuropharmaceutical agent conjugate when the antibody is reactive with a transferrin receptor, for the manufacture of a medicament for delivering the neuropharmaceutical agent across the blood brain barrier to the brain of a host, under conditions whereby binding of the antibody to transferrin receptors present on brain capillary endothelial cells occurs and the neuropharmaceutical agent is transferred across the blood brain barrier in a pharmaceutically active form.

2. Use according to claim 1, wherein the neuropharmaceutical agent is (a) a protein, e.g. CD4, tricosanthin, interferon, nerve growth factor or dopamine decarboxylase; or (b) an antibiotic, e.g. amphotericin B, gentamycin or pyrimethamine; or (c) an adrenergic agent, e.g. dopamine or atenolol; or (d) a nucleotide analog, e.g. azido thymidine, dideoxy Inosine or dideoxy cytodine; or (e) a chemotherapeutic agent, selected e.g. from methotrexate, cyclophosphamide, etoposide, carboplatin and adriamycin; or (f) an anticonvulsant, e.g. valproate; or (g) an anti-trauma agent, e.g. superoxide dimutase; or (h) a peptide, e.g. a somatotostatin analogue or an enkephalinase inhibitor.

3. Use according to claim 1, wherein the antibody is a monoclonal antibody, selected from e.g. OX-26, B3/25, T56/14, OKT9, L5.1, 5E-9, R17 217 and T58/30.

4. Use according to claim 1, wherein the neuropharmaceutical agent is linked to the antibody via a cleavable link, and wherein for example the cleavable link is formed using cis-aconitic acid, cis-carboxylic alkatriene or poly-maleic anhydride.

5. Use according to claim 4, wherein the neuropharmaceutical agent is selected from azido thymidine, dideoxy Inosine, dideoxy cytodine, adriamycin, amphotericin B, pyrimethamine, valproate, methotrexate, cyclophosphamide, carboplatin, gentamycin, dopamine, atenolol and etoposide.

6. Use according to claim 1, wherein the neuropharmaceutical agent is linked to the antibody via a noncleavable link, e.g. an amide bond, a bond between a sulfhydryl group and a maleimide derivative, a bond between a thiol and an amino group or a bond between an aldehyde and an amino group.

7. Use according to claim 6, wherein the neuropharmaceutical agent is a protein or peptide.

8. A conjugate for delivering a neuropharmaceutical agent across the blood brain barrier comprising an antibody reactive with a transferrin receptor linked to a growth factor as the neuropharmaceutical agent, whereby the conjugate transports the growth factor neuropharmaceutical agent across the blood brain barrier when administered in vivo.

9. A conjugate for delivering a neuropharmaceutical agent across the blood brain barrier comprising an antibody reactive with a transferrin receptor linked to a nucleotide analog as the pharmaceutical agent, whereby the conjugate transports the nucleotide analog pharmaceutical agent across the blood brain barrier when administered in vivo.

10. Use of an antibody-neuropharmaceutical agent conjugate wherein the antibody is reactive with a transferrin receptor, for the manufacture of a medicament for treating a host afflicted with a disease associated with a neurological disorder, under conditions whereby binding of the antibody to the transferrin receptor present on a brain capillary endothelial cell occurs and the neuropharmaceutical agent is transferred across the blood brain barrier in a pharmaceutically active form.

11. Use of a ligand-neuropharmaceutical agent conjugate wherein the ligand is reactive with a transferrin receptor, for the manufacture of a medicament for delivering a neuropharmaceutical agent across the blood brain barrier to the brain of a host under conditions whereby binding of the ligand to transferrin receptors present on brain capillary endothelial cells occurs and the neuropharmaceutical agent is transferred across the blood brain barrier in a pharmaceutically active form.

## Claims (Claims for the following Contracting State(s): ES, GR)

1. Use of an antibody-neuropharmaceutical agent conjugate when the antibody is reactive with a transferrin receptor, for the manufacture of a medicament for delivering the neuropharmaceutical agent across the blood brain barrier to the brain of a host, under conditions whereby binding of the antibody to transferrin receptors present on brain capillary endothelial cells occurs and the neuropharmaceutical agent is transferred across the blood brain barrier in a pharmaceutically active form.

2. Use according to claim 1, wherein the neuropharmaceutical agent is (a) a protein, e.g. CD4, tricosanthin, interferon, nerve growth factor or dopamine decarboxylase; or (b) an antibiotic, e.g. amphotericin B, gentamycin or pyrimethamine; or (c) an adrenergic agent, e.g. dopamine or atenolol; or (d) a nucleotide analog, e.g. azido thymidine, dideoxy Inosine or dideoxy cytodine; or (e) a chemotherapeutic agent, selected e.g. from methotrexate, cyclophosphamide, etoposide, carboplatin and adriamycin; or (f) an anticonvulsant, e.g. valproate; or (g) an anti-trauma agent, e.g. superoxide dimutase; or (h) a peptide, e.g. a somatotostatin analogue or an enkephalinase inhibitor.

3. Use according to claim 1, wherein the antibody is a monoclonal antibody, selected from e.g. OX-26, B3/25, T56/14, OKT9, L5.1, 5E-9, R17 217 and T58/30.

4. Use according to claim 1, wherein the neuropharmaceutical agent is linked to the antibody via a cleavable link, and wherein for example the cleavable link is formed using cis-aconitic acid, cis-carboxylic alkatriene or poly-maleic anhydride.

5. Use according to claim 4, wherein the neuropharmaceutical agent is selected from azido thymidine, dideoxy Inosine, dideoxy cytodine, adriamycin, amphotericin B, pyrimethamine, valproate, methotrexate, cyclophosphamide, carboplatin, gentamycin, dopamine, atenolol and etoposide.

6. Use according to claim 1, wherein the neuropharmaceutical agent is linked to the antibody via a noncleavable link, e.g. an amide bond, a bond between a sulfhydryl group and a maleimide derivative, a bond between a thiol and an amino group or a bond between an aldehyde and an amino group.

7. Use according to claim 6, wherein the neuropharmaceutical agent is a protein or peptide.

8. A method of making a conjugate for delivering a neuropharmaceutical agent across the blood brain barrier comprising linking an antibody reactive with a transferrin receptor to a growth factor as the neuropharmaceutical agent, whereby the conjugate transports the growth factor neuropharmaceutical agent across the blood brain barrier when administered in vivo.

9. A method of making a conjugate for delivering a neuropharmaceutical agent across the blood brain barrier comprising linking an antibody reactive with a transferrin receptor to a nucleotide analog as the pharmaceutical agent, whereby the conjugate transports the nucleotide analog pharmaceutical agent across the blood brain barrier when administered in vivo.

10. Use of an antibody-neuropharmaceutical agent conjugate wherein the antibody is reactive with a transferrin receptor, for the manufacture of a medicament for treating a host afflicted with a disease associated with a neurological disorder, under conditions whereby binding of the antibody to the transferrin receptor present on a brain capillary endothelial cell occurs and the neuropharmaceutical agent is transferred across the blood brain barrier in a pharmaceutically active form.

11. Use of a ligand-neuropharmaceutical agent conjugate wherein the ligand is reactive with a transferrin receptor, for the manufacture of a medicament for delivering a neuropharmaceutical agent across the blood brain barrier to the brain of a host under conditions whereby binding of the ligand to transferrin receptors present on brain capillary endothelial cells occurs and the neuropharmaceutical agent is transferred across the blood brain barrier in a pharmaceutically active form.

## Patentansprüche (Patentansprüche für folgende(n) Vertragsstaat(en): AT, BE, CH, DE, DK, FR, GB, IT, LI, LU, NL, SE)

1. Verwendung eines Antikörper-neuropharmazeutischen Mittel-Konjugats, wenn der Antikörper mit einem Transferrinrezeptor reagiert, zur Herstellung eines Medikaments zum Transport des neuropharmazeutischen Mittels durch die Blut-Hirn-Schranke zum Gehirn eines Patienten unter Bedingungen, wo ein Binden des Antikörpers an Transferrinrezeptoren erfolgt, die sich auf Endothelzellen der Gehirnkapillaren befinden, und das neuropharmazeutische Mittel durch die Blut-Hirn-Schranke in einer pharmazeutisch aktiven Form transportiert wird.

2. Verwendung gemäß Anspruch 1, worin das neuropharmazeutische Mittel (a) ein Protein, z. B. CD4, Tricosanthin, Interferon, ein Nervenwachstumsfaktor oder Dopamindecarboxylase; oder (b) ein Antibiotikum, z. B. Amphotericin B, Gentamycin oder Pyrimethamin; oder (c) ein adrenerges Mittel, z. B. Dopamin oder Atenolol; oder (d) ein Nucleotid-Analogon, z. B. Azidothymidin, Dideoxyinosin oder Dideoxycytodin; oder (e) ein Chemotherapeutikum, ausgewählt aus z. B. Methotrexat, Cyclophosphamid, Etoposid, Carboplatin und Adriamycin; oder (f) ein Antikonvulsivum, z. B. Valproat; oder (g) ein Antitrauma-Mittel, z. B. Superoxiddismutase; oder (h) ein Peptid, z. B. ein Somatotostatin-Analogon oder ein Enkephalinase-Inhibitor, ist.

3. Verwendung gemaß Anspruch 1, worin der Antikörper ein monoklonaler Antikörper, ausgewählt aus z. B. OX-26, B3/25, T56/14, OKT9, L5.1, 5E-9, R17 217 und T58/30, ist.

4. Verwendung gemäß Anspruch 1, worin das neuropharmazeutische Mittel an den Antikörper über eine spaltbare Bindung gebunden ist, und worin zum Beispiel die spaltbare Bindung durch Verwendung von cis-Aconitsäure, cis-Carboxylalkatrien oder Polymaleinsäureanhydrid gebildet wird.

5. Verwendung gemäß Anspruch 4, worin das neuropharmazeutische Mittel ausgewählt aus Azidothymidin, Dideoxyinosin, Dideoxycytodin, Adriamycin, Amphotericin B, Pyrimethamin, Valproat, Methotrexat, Cyclophosphamid, Carboplatin, Gentamycin, Dopamin, Atenolol und Etoposid ist.

6. Verwendung gemäß Anspruch 1, worin das neuropharmazeutische Mittel an den Antikörper über eine nichtspaltbare Bindung gebunden ist, z. B. eine Amidbindung, eine Bindung zwischen einer Sulfhydrylgruppe und einem Maleimidderivat, eine Bindung zwischen einer Thiol- und einer Aminogruppe oder eine Bindung zwischen einer Aldehyd- und einer Aminogruppe.

7. Verwendung gemaß Anspruch 1, worin das neuropharmazeutische Mittel ein Protein oder Peptid ist.

8. Ein Konjugat, das einen Antikörper aufweist, der mit einem Transferrinrezeptor reagiert und an einen Wachstumsfaktor als das neuropharmazeutische Mittel gebunden ist, zum Transport eines neuropharmazeutischen Mittels durch die Blut-Hirn-Schranke, wodurch das Konjugat den Wachstumsfaktor, ein neuropharmazeutisches Mittel, durch die Blut-Hirn-Schranke transportiert, wenn es in vivo verabreicht wird.

9. Ein Konjugat, das einen Antikörper aufweist, der mit einem Transferrinrezeptor reagiert und an ein Nucleotid-Analogon als das pharmazeutische Mittel gebunden ist, zum Transport eines neuropharmazeutischen Mittels durch die Blut-Hirn-Schranke, wodurch das Konjugat das Nucleotid-Analogon, ein pharmazeutisches Mittel, durch die Blut-Hirn-Schranke transportiert, wenn es in vivo verbreicht wird.

10. Verwendung eines antikörper-neuropharmazeutischen Mittel-Konjugats, worin der Antikörper mit einem Transferrinrezeptor reagiert, zur Herstellung eines Medikaments zur Behandlung eines Patienten, der an einer Krankheit leidet, die mit einer neurologischen Störung verbunden ist, unter Bedingungen, wo ein Binden des Antikörpers an den Transferrinrezeptor erfolgt, der sich auf einer Endothelzelle der Gehirnkapillaren befindet, und das neuropharmazeutische Mittel in einer pharmazeutisch aktiven Form durch die Blut-Hirn-Schranke transportiert wird.

11. Verwendung eines ligand-pharmazeutischen Mittel-Konjugats, worin der Ligand mit einem Transferrinrezeptor reagiert, zur Herstellung eines Medikaments zum Transport eines neuropharmazeutischen Mittels durch die Blut-Hirn-Schranke zum Gehirn eines Patienten unter Bedingungen, wo ein Binden des Liganden an Transferrinrezeptoren erfolgt, die sich auf Endothelzellen der Gehirnkapillaren befinden, und das neuropharmazeutische Mittel durch die Blut-Hirn-Schranke in einer pharmazeutisch aktiven Form transportiert wird.

## Patentansprüche (Patentansprüche für folgende(n) Vertragsstaat(en): ES, GR)

1. Verwendung eines antikörper-neuropharmazeutischen Mittel-Konjugats, wenn der Antikörper mit einem Transferrinrezeptor reagiert, zur Herstellung eines Medikaments zum Transport des neuropharmazeutischen Mittels durch die Blut-Hirn-Schranke zum Gehirn eines Patienten unter Bedingungen, wo ein Binden des Antikörpers an Transferrinrezeptoren erfolgt, die sich auf Endothelzellen der Gehirnkapillaren befinden, und das neuropharmazeutische Mittel durch die Blut-Hirn-Schranke in einer pharmazeutisch aktiven Form transportiert wird.

2. Verwendung gemäß Anspruch 1, worin das neuropharmazeutische Mittel (a) ein Protein, z. B. CD4, Tricosanthin, Interferon, ein Nervenwachstumsfaktor oder Dopamindecarborylase; oder (b) ein Antibiotikum, z. B. Amphotericin B, Gentamycin oder Pyrimethamin; oder (c) ein adrenerges Mittel, z. B. Dopamin oder Atenolol; oder (d) ein Nucleotid-Analogon, z. B. Azidothymidin, Dideoxyinosin oder Dideoxycytodin; oder (e) ein Chemotherapeutikum, ausgewählt aus z. B. Methotrexat, Cyclophosphamid, Etoposid, Carboplatin und Adriamycin; oder (f) ein Antikonvulsivum, z. B. Valproat; oder (g) ein Antitrauma-Mittel, z. B. Superoxiddismutase; oder (h) ein Peptid, z. B. ein Somatotostatin-Analogon oder ein Enkephalinase-Inhibitor, ist.

3. Verwendung gemäß Anspruch 1, worin der Antikörper ein monoklonaler Antikörper, ausgewählt aus z. B. OX-26, B3/25, T56/14, OKT9, L5.1, 5E-9, R17 217 und T58/30, ist.

4. Verwendung gemäß Anspruch 1, worin das neuropharmazeutische Mittel an den Antikörper über eine spaltbare Bindung gebunden ist, und worin zum Beispiel die spaltbare Bindung durch Verwendung von cis-Aconitsäure, cis-Carboxylalkatrien oder Polymaleinsäureanhydrid gebildet wird.

5. Verwendung gemäß Anspruch 4, worin das neuropharmazeutische Mittel ausgewählt aus Azidothymidin, Dideoxyinosin, Dideoxycytodin, Adriamycin, Amphotericin B, Pyrimethamin, Valproat, Methotrexat, Cyclophosphamid, Carboplatin, Gentamycin, Dopamin, Atenolol und Etoposid ist.

6. Verwendung gemäß Anspruch 1, worin das neuropharmazeutische Mittel an den Antikörper über eine nichtspaltbare Bindung gebunden ist, z. B. eine Amidbindung, eine Bindung zwischen einer Sulfhydrylgruppe und einem Maleimidderivat, eine Bindung zwischen einer Thiol- und einer Aminogruppe oder eine Bindung zwischen einer Aldehyd- und einer Aminogruppe.

7. Verwendung gemäß Anspruch 1, worin das neuropharmazeutische Mittel ein Protein oder Peptid ist.

8. Ein Verfahren zur Herstellung eines Konjugats zum Transport eines neuropharmazeutischen Mittels durch die Blut-Hirn-Schranke, wobei das Verfahren ein Binden eines mit einem Transferrinrezeptor reagierenden Antikörpers an einen Wachstumsfaktor als das neuropharmazeutische Mittel umfaßt, wodurch das Konjugat den Wachstumsfaktor, ein neuropharmazeutisches Mittel, durch die Blut-Hirn-Schranke transportiert, wenn es in vivo verabreicht wird.

9. Ein Verfahren zur Herstellung eines Konjugats zum Transport eines neuropharmazeutischen Mittels durch die Blut-Hirn-Schranke, wobei das Verfahren ein Binden eines mit einem Transferrinrezeptor reagierenden Antikörpers an ein Nucleotid-Analogon als das pharmazeutische Mittel umfaßt, wodurch das Konjugat das Nucleotid-Analogon, ein pharmazeutisches Mittel, durch die Blut-Hirn-Schranke transportiert, wenn es in vivo verbreicht wird.

10. Verwendung eines antikörper-neuropharmazeutischen Mittel-Konjugats, worin der Antikörper mit einem Transferrinrezeptor reagiert, zur Herstellung eines Medikaments zur Behandlung eines Patienten, der an einer Krankheit leidet, die mit einer neurologischen Störung verbunden ist, unter Bedingungen, wo ein Binden des Antikörpers an den Transferrinrezeptor erfolgt, der sich auf einer Endothezelle der Gehirnkapillaren befindet, und das neuropharmazeutische Mittel in einer pharmazeutisch aktiven Form durch die Blut-Hirn-Schranke transportiert wird.

11. Verwendung eines ligand-pharmazeutischen Mittel-Konjugats, worin der Ligand mit einem Transferrinrezeptor reagiert, zur Herstellung eines Medikaments zum Transport eines neuropharmazeutischen Mittels durch die Blut-Hirn-Schranke zum Gehirn eines Patienten unter Bedingungen, wo ein Binden des Liganden an Transferrinrezeptoren erfolgt, die sich auf Endothelzellen der Gehirnkapillaren befinden, und das neuropharmazeutische Mittel durch die Blut-Hirn-Schranke in einer pharmazeutisch aktiven Form transportiert wird.

## Revendications (Revendications pour l'(les) Etat(s) contractant(s) suivant(s): AT, BE, CH, DE, DK, FR, GB, IT, LI, LU, NL, SE)

1. Utilisation d'un conjugué anticorps-agent neuropharmaceutique dans lequel l'anticorps réagit avec un récepteur de la transférine, pour la fabrication d'un médicament pour libérer l'agent neuropharmaceutique à travers la barrière hémato-encéphalique dans le cerveau de l'hote, selon des conditions telles que l'anticorps se fixe sur les récepteurs de la transférine présents sur les cellules endothéliales des capillaires du cerveau et l'agent neuropharmaceutique est transporté à travers la barrière hémato-encéphalique sous une forme pharmaceutiquement active.

2. Utilisation selon la revendication 1,dans laquelle l'agent neuropharmaceutique est : (a) une protéine, exemple le CD4, la tricosanthine, l'interféron, le facteur de croissance nerveuse ou la dopamine décarboxylase ; ou (b) un antibiotique, ex. l'amphotéricine B, la gentamycine ou la pyriméthamine ; ou (c) un agent adrénergique, ex. la dopamine ou l'aténolol ; ou (d) un analogue de nucléotide, ex. l'azido thymidine, la didesoxy inosine ou la didesoxy cytidine ; ou (e) un agent chimiothérapeutique, choisi parmi par exemple le méthotréxate, la cyclophosphamide, l'étoposide, le carboplatine et l'adriamycine ; ou (f) un antiépileptique, ex. le valproate ; ou (g) un agent anti-traumatisme ex. la superoxyde dismutase ; ou (h) un peptide, ex. un analogue de la somatotostatine ou un inhibiteur de l'enkephalinase.

3. Utilisation selon la revendication 1, dans laquelle l'anticorps est un anticorps monoclonal choisi parmi par exemple OX-26, B3/25, T56/14, OKT9, L5.1, 5E-9, R17 217 and T58/30.

4. Utilisation selon la revendication 1, dans laquelle l'agent neuropharmaceutique est lié à l'anticorps par une liaison clivable et dans laquelle par exemple la liaison clivable est formée en utilisant un acide cis-aconitique, un acide cis-alcatriène carboxylique un poly anhydride maléïque.

5. Utilisation selon la revendication 4, dans laquelle l'agent neuropharmaceutique est choisi parmi l'azido thymidine, la didesoxy inosine, la didesoxy cytidine, l'adriamycine, l'amphotéricine B, la pyriméthamine, le valproate, le méthotrexate, la cyclophosphamide, le carboplatine, la gentamycine, la dopamine, l'aténolol et l'étoposide.

6. Utilisation selon la revendication 1, dans laquelle l'agent neuropharmaceutique est lié à l'anticorps par une liaison non clivable par exemple une liaison amide, une liaison entre un groupe sulfhydryle et un dérivé maléimide, une liaison entre un thiol et un groupe amine ou une liaison entre un aldéhyde et un groupe amine.

7. Utilisation selon la revendication 6, dans laquelle l'agent neuropharmaceutique est une protéine ou un peptide.

8. Conjugué pour la libération d'un agent neuropharmaceutique à travers la barrière hémato-encéphalique comprenant un anticorps qui réagit avec un récepteur de la transférine lié à un facteur de croissance en tant qu'agent neuropharmaceutique, où le conjugué transporte le facteur de croissance agent neuropharmaceutique à travers la barrière hémato-encéphalique lorsqu'il est administré in vivo.

9. Conjugué pour la libération d'un agent neuropharmaceutique à travers la barrière hémato-encéphalique comprenant un anticorps qui réagit avec un récepteur de la transférine lié à un analogue de nucléotide en tant qu'agent pharmaceutique, où le conjugué transporte l'analogue de nucléotide agent pharmaceutique à travers la barrière hémato-encéphalique lorsqu'il est administré in vivo.

10. Utilisation d'un conjugué anticorps-agent neuropharmaceutique dans lequel l'anticorps réagit avec un récepteur de la transférine, pour la fabrication d'un médicament pour traiter un hote atteint d'une affection associée à un désordre neurologique selon des conditions telles que l'anticorps se fixe sur le récepteur de la transférine présent sur les cellules andothéliales des capillaires du cerveau et l'agent neuropharmaceutique est transporté à travers la barrière hématoencéphalique sous une forme pharmaceutiquement active.

11. Utilisation d'un conjugué ligand-agent neuropharmaceutique dans lequel le ligand réagit avec un récepteur de la transférine, pour la fabrication d'un médicament pour délivrer l'agent neuropharmaceutique à travers la barrière hémato-encéphalique vers le cerveau de l'hote, selon des conditions telles que le ligand se fixe sur les récepteurs de la transférine présents sur les cellules endothéliales des capillaires du cerveau et l'agent neuropharmaceutique est transporté à travers la barrière hémato-encéphalique sous une forme pharmaceutiquement active.

## Revendications (Revendications pour l'(les) Etat(s) contractant(s) suivant(s): ES, GR)

1. Utilisation d'un conjugué anticorps-agent neuropharmaceutique dans lequel l'anticorps réagit avec un récepteur de la transférine, pour la fabrication d'un médicament pour la délivrance de l'agent neuropharmaceutique à travers la barrière hémato-encéphalique dans le cerveau de l'hote, selon des conditions telles que l'anticorps se fixe sur les récepteurs de la transférine présents sur les cellules endothéliales des capillaires du cerveau et l'agent neuropharmaceutique est transporté à travers la barrière hémato-encéphalique sous une forme pharmaceutiquement active.

2. Utilisation selon la revendication 1,dans laquelle l'agent neurophamarceutique est :(a) une protéine, exemple le CD4, la tricosanthine, l'interféron, le facteur de croissance nerveuse ou la dopamine décarboxylase ; ou (b) un antibiotique, ex. l'amphotéricine B, la gentamycine ou la pyriméthamine ; ou (c) un agent adrénergique, ex. la dopamine ou l'aténolol ; ou (d) un analogue de nucléotide, ex. l'azido thymidine, la didesoxy inosine ou la didesoxy cytidine ; ou (e) un agent chimiothérapeutique, choisi parmi par exemple le méthotréxate, la cyclophosphamide, l'étoposide, le carboplatine et l'adriamycine ; ou (f) un antiépileptique, ex. le valproate ; ou (g) un agent anti-traumatisme ex. la superoxyde dismutase ; ou (h) un peptide, ex. un analogue de la somatotostatine ou un inhibiteur de l'enkephalinase.

3. Utilisation selon la revendication 1, dans laquelle l'anticorps est un anticorps monoclonal choisi parmi par exemple OX-26, B3/25, T56/14, OKT9, L5.1, 5E-9, R17 217 and T58/30.

4. Utilisation selon la revendication 1, dans laquelle l'agent neuropharmaceutique est lié à l'anticorps par une liaison clivable et dans laquelle par exemple la liaison clivable est formée en utilisant un acide cis-aconitique, un acide cis-alcatriène carboxylique un poly anhydride maléïque .

5. Utilisation selon la revendication 4, dans laquelle l'agent neuropharmaceutique est choisi parmi l'azido thymidine, la didesoxy inosine, la didesoxy cytidine, l'adriamycine, l'amphotéricine B, le pyriméthamine, le valproate, le méthotrexate, la cyclophosphamide, le carboplatine, la gentamycine, la dopamine, l'aténolol et l'étoposide.

6. Utilisation selon la revendication 1, dans laquelle l'agent neuropharmaceutique est lié à l'anticorps par une liaison non clivable par exemple une liaison amide, une liaison entre un groupe sulfhydryle et un dérivé maléimide, une liaison entre un thiol et un groupe amine ou une liaison entre un aldéhyde et un groupe amine.

7. Utilisation selon la revendication 6, dans laquelle l'agent neuropharmaceutique est une protéine ou un peptide.

8. Méthode de fabrication d'un conjugué pour la délivrance d'un agent neuropharmaceutique à travers la barrière hémato-encéphalique comprenant la liaison d'un anticorps réactif réagissant avec un récepteur de la transférine à un facteur de croissance en tant qu'agent neuropharmaceutique, où le conjugué transporte le facteur de croissance agent neuropharmaceutique à travers la barrière hémato-encéphalique lorsqu'il est administré in vivo.

9. Méthode de fabrication d'un conjugué pour la délivrance d'un agent neuropharmaceutique à travers la barrière hémato-encéphalique comprenant la liaison d'un anticorps réactif réagissant avec un récepteur de la transférine à un analogue de nucléotide en tant qu'agent pnarmaceutique, où le conjugué transporte l'analogue de nucléotide agent pharmaceutique à travers la barrière hémato-encéphalique lorsqu'il est administré in vivo.

10. Utilisation d'un conjugué anticorps-agent neuropharmaceutique dans lequel l'anticorps réagit avec un récepteur de la transférine, pour la fabrication d'un médicament pour traiter un hote atteint d'une affection associée à un désordre neurologique selon des conditions telles que l'anticorps sur le récepteur de la transférine présent sur les cellules andothéliales des capillaires du cerveau et l'agent neuropharmaceutique est transporté à travers la barrière hémato-encéphalique sous une forme pharmaceutiquement active.

11. Utilisation d'un conjugué ligand-agent neuropharmaceutique dans lequel le ligand réagit avec un récepteur de la transférine, pour la fabrication d'un médicament pour libérer l'agent neuropharmaceutique à travers la barrière hémato-encéphalique vers le cerveau de l'hote, selon des conditions telles que le ligand se fixe sur les récepteurs de la transférine présents sur les cellules endothéliales des capillaires du cerveau et l'agent neuropharmaceutique est transporté à travers la barrière hémato-encéphalique sous une forme pharmaceutiquement active.
